# EUROPÄISCHE PATENTANMELDUNG

(11) **EP 4 173 650 A1**
(43) Veröffentlichungstag der Anmeldung: **03.05.2023**
(21) Anmeldenummer: 22203346.6
(22) Anmeldetag: 24.10.2022
(51) Int. Cl.: A61M 5/168

(54) **INFUSIONSSYSTEM**

(30) Priorität: 28.10.2021 DE 102021212211
(71) Anmelder: B. Braun Melsungen AG, 34212 Melsungen (DE)
(72) Erfinder: Moritzen, Hans-Christian, 34119 Kassel (DE); Obermann, Dennis, 34628 Willingshausen (DE); Wiegand, Thomas, 34576 Homberg (DE)
(74) Vertreter: Patentanwälte Ruff, Wilhelm, Beier, Dauster & Partner mbB

(57) **Zusammenfassung**

Infusionssystem (1), aufweisend:
- eine Infusionspumpe (2), die dazu ausgebildet ist, eine zu verabreichende Flüssigkeit (3) in einer Flüssigkeitsleitung (4) zu fördern,
- eine Anzahl von Sensoren (5, 6, 7, 8) zur Erzeugung zugehöriger Sensordaten (S1, S2, S3, S4), und
- eine KI-Einheit (9), die dazu ausgebildet ist, eine Anzahl von Zielgrößen (M1, M2, M3) in Abhängigkeit von der Anzahl von Sensordaten (S1, S2, S3, S4) basierend auf einem statistischen Modell zu berechnen, wobei das statistische Modell mittels Trainingsdaten trainiert worden ist.

## Beschreibung

Der Erfindung liegt die Aufgabe zugrunde, ein Infusionssystem zur Verfügung zu stellen, das eine hohe Betriebssicherheit aufweist.

Das erfindungsgemäße Infusionssystem weist eine herkömmliche Infusionspumpe auf, die dazu ausgebildet ist, eine zu verabreichende Flüssigkeit in einer Flüssigkeitsleitung zu fördern. Die Infusionspumpe kann beispielsweise eine Spritzenpumpe oder eine Schlauchpumpe sein.

Das Infusionssystem weist weiter eine Anzahl von Sensoren zur Erzeugung zugehöriger Sensordaten auf. Die Anzahl von Sensoren kann beispielsweise zwischen 1 und 20 betragen.

Das Infusionssystem weist weiter eine KI-Einheit auf, die dazu ausgebildet ist, eine Anzahl von Zielgrößen in Abhängigkeit von der Anzahl von Sensordaten basierend auf einem statistischen Modell zu berechnen, wobei das statistische Modell mittels Trainingsdaten trainiert worden ist. Die Anzahl von Zielgrößen kann beispielsweise zwischen 1 und 10 betragen.

In einer Ausführungsform ist die Anzahl von Sensoren ausgewählt aus der Menge von Sensoren bestehend aus: Drucksensoren, die dazu ausgebildet sind, Sensordaten in Form von Druckdaten der zu verabreichenden Flüssigkeit in der Flüssigkeitsleitung zu erzeugen, Kraftsensoren, die dazu ausgebildet sind, Sensordaten in Form von Kraftdaten einer mittels der Infusionspumpe erzeugten Pumpkraft zu erzeugen, Motorstromsensoren, die dazu ausgebildet sind, Sensordaten in Form von Motorstromdaten eines elektrischen Antriebsmotors der Infusionspumpe zu erzeugen, und Positionssensoren, die dazu ausgebildet sind, Sensordaten in Form von Positionsdaten eines Spritzenkolbens der Infusionspumpe zu erzeugen.

In einer Ausführungsform ist die Anzahl von Zielgrößen ausgewählt aus der Menge von Zielgrößen bestehend aus: einem absoluten Druck in der Flüssigkeitsleitung, einer Wahrscheinlichkeit eines Verschlusses der Flüssigkeitsleitung und einem Ort des Verschlusses der Flüssigkeitsleitung.

In einer Ausführungsform ist die KI-Einheit dazu ausgebildet, die Anzahl von Zielgrößen weiter in Abhängigkeit von Medikamentendaten, Patientendaten, Daten von weiteren Infusionspumpen, Therapiedaten, Umgebungsbedingungsdaten und/oder Vitalparameterdaten basierend auf dem statistischen Modell zu berechnen.

In einer Ausführungsform weist das Infusionssystem weiter eine Alarmeinheit auf, die dazu ausgebildet ist, basierend auf den Zielgrößen zu überprüfen, ob eine Alarmbedingung erfüllt ist, und einen Alarm zu erzeugen, falls die Alarmbedingung erfüllt ist.

In einer Ausführungsform weist das Infusionssystem weiter eine Korrelationsanalyseeinheit auf, die dazu ausgebildet ist, basierend auf allen oder einem Teil der Sensordaten und/oder basierend auf allen oder einem Teil der Zielgrößen Gewichtungsfaktoren zu berechnen, wobei die Alarmeinheit dazu ausgebildet ist, basierend auf den Gewichtungsfaktoren zu überprüfen, ob die Alarmbedingung erfüllt ist.

In einer Ausführungsform ist die Alarmeinheit dazu ausgebildet, die Infusionspumpe beim Eintreten der Alarmbedingung zu stoppen.

Ziel einer Infusionstherapie ist eine kontinuierliche, über eine bestimmte Zeit ablaufende Verabreichung von Flüssigkeiten. Dies geschieht in der Regel intravenös, arteriell, subkutan oder intraossär über einen Katheter. Daneben gibt es noch die parenterale Ernährung mittels Infusionspumpen.

Für eine hohe Dosiergenauigkeit werden in der apparativen Infusionstechnik Infusionspumpen verwendet. Bekannte Ausprägungen von Infusionspumpen sind beispielsweise Spritzenpumpen sowie Schlauchpumpen. Während der Anwendung mit diesen Pumpen kann es zu verschiedenen Betriebsstörungen kommen. Ein Beispiel ist der Verschluss der Flüssigkeitsleitung und/oder des Patientenzugangs. Diese Betriebsstörung wird auch als Okklusion bezeichnet und führt zu einer Unterbrechung der Förderung. Da dies zu einer erheblichen Patientengefährdung führen kann, ist die Detektion eines Verschlusses fester Funktionsumfang herkömmlicher Infusionsgeräte.

Herkömmliche Infusionssysteme verwenden üblicherweise feste Schwellwerte, um einen Verschluss zu detektieren.

Bei kleinsten Infusionsraten kann es hierbei jedoch mehrere Stunden dauern, bis ein Verschluss erkannt wird. Die frühe Erkennung eines Verschlusses kann aber bei diesen niedrigen Raten besonders relevant sein, da häufig hochwirksame Medikamente mit diesen Raten verabreicht werden.

Die Kombination aus niedrigen Raten und gleichzeitig hochpotenten Medikamenten ist bei den bisherigen auf dem Markt verfügbaren Infusionsgeräten im Fall eines Verschlusses als kritisch zu betrachten.

Ein Problem besteht darin, dass der Einsatz kleinster Schwellwerte sich bei den konventionellen Methoden besonders schwierig gestaltet, da diese mit einer hohen Fehlalarmquote einhergehen. Grund sind verschiedene Störgrößen, welche das relevante Sensorsignal (Druck/Kraft) überlagern, wie beispielsweise der Übergang von Haft- zur Gleitreibung bei einer Spritzenpumpe (Slip-Stick-Effekt) sowie generelle Schwankungen in der Beschaffenheit der Kunststoffeinmalartikel.

Erfindungsgemäß werden mittels der KI-Einheit die relevanten Sensordaten, beispielswiese betreffend einen mittels der Pumpe erzeugten Druck und/oder eine mittels der Pumpe erzeugte Pump-Kraft, und gegebenenfalls weitere Daten bzw. Eingangsgrößen ausgewertet, die auf einen Verschluss schließen lassen. Erfindungsgemäß werden diese Eingangsgrößen über eine Mustererkennung zur Verschlusserkennung ausgewertet. Die KI-Einheit kann integrierter Bestandteil der Infusionspumpe sein. Die KI-Einheit kann einen nicht-selbstlernenden KI-Algorithmus ausführen.

Die KI-Einheit ermittelt bevorzugt drei Zielgrößen zur Verschlusserkennung, nämlich einen absoluten bzw. realen Druck in der Flüssigkeitsleitung, eine Wahrscheinlichkeit eines Verschlusses und einen Ort des Verschlusses.

Die Erfindung ermöglicht es zunächst, dass aus den zu verarbeitenden Sensordaten, betreffend beispielsweise einen gemessenen Druck bzw. eine gemessene Kraft, eine erste Zielgröße in Form eines realen Flüssigkeitsdrucks in der Flüssigkeitsleitung ermittelt werden kann, wobei in Abhängigkeit von dem realen Flüssigkeitsdruck ein Alarm erzeugt werden kann. Es werden Störungen in den Sensordaten reduziert, wodurch Fehlalarme erkannt und minimiert werden können.

Die Erfindung ermöglicht weiter das Ermitteln einer zweiten Zielgröße in Form eines Trends bzw. einer Wahrscheinlichkeit eines Verschlusses. Da bei einigen Anwendungsfällen ein Mindestdruck benötigt wird, ist neben dem absoluten Druck in der Flüssigkeitsleitung auch die Wahrscheinlichkeit eines Verschlusses von Relevanz. Erfindungsgemäß wird anhand der Sensordaten bzw. Eingangsgrößen die Wahrscheinlichkeit eines Verschlusses ermittelt. Ein Druckanstieg aufgrund eines Verschlusses folgt einem bestimmten Muster in den Sensordaten. Dieses Muster wird mittels der KI-Einheit erkannt, um mit Hilfe einer Trendanalyse auch bei kleinen Förderraten einen Verschluss frühzeitig erkennen zu können. Bei Verwendung herkömmlicher Schwellwertsystemen können mehrere Stunden vergehen, bis ein Verschluss erkannt wird und ein entsprechender Alarm ausgelöst wird.

Die Unterscheidung zum absoluten Druck bzw. zur ersten Zielgröße kann außerdem sinnvoll sein, wenn ein hoher Druck in der Anwendung keine Patientengefährdung erzeugt und trotzdem eine zügige Alarmreaktion erwünscht ist. Konkret können zur Unterscheidung eines Verschlusses von anderen Einflussfaktoren pumpenseitig die Sensordaten jeweils in Ruhe und während bzw. kurz nach Ausführung eines Pumpen-Motorschrittes erhoben werden. Die KI-Einheit wird damit in die Lage versetzt, einen echten Verschluss der Flüssigkeitsleitung von anderen Störgrößen zu unterscheiden, wie zum Beispiel die Umlagerung des Patienten.

Die Erfindung ermöglicht weiter das Ermitteln einer dritten Zielgröße in Form eines Ortes des Verschlusses. Dem liegt die erfindungsgemäße Erkenntnis zugrunde, dass der Ort des Verschlusses, der beispielsweise eine Entfernung von der Kraftmessung bis zum Verschluss beschreibt, ein ortsspezifisches Muster in den Sensordaten bewirkt.

Die Zielgrößen werden der Alarmeinheit zugeführt, so dass Alarme, Voralarme oder Hinweise für den Anwender generiert werden können.

Die KI-Einheit verwendet statistische Modelle und wird über das Anlernen anhand von Daten trainiert. Dies wird als "Supervised Learning" bezeichnet. Dazu benötigt man reale Sensordaten bzw. Eingangsdaten, die mit den Zielgrößen, d.h. beispielsweise "Verschluss ja/nein" und dem realen Flüssigkeitsdruck, verknüpft sind. D.h. die Zielgrößen sind zu den jeweiligen Sensordaten bzw. Eingangsgrößen bekannt. Weitere Sensordaten bzw. Eingangsdaten und deren korrelierenden Zielgrößen können durch physikalische Simulationen erzeugt werden. Die KI-Einheit lernt mit diesen Daten und kann ein Vorhersagemodell erstellen.

Um das System anschließend validieren zu können und auch zu prüfen, ob das System die notwendige Vorhersagegenauigkeit aufweist, können die Daten in Trainingsdaten und Validierungsdaten aufgeteilt werden. Mit den Trainingsdaten wird die KI-Einheit angelernt und mit den Validierungsdaten wird anschließend getestet.

Die Erfindung wird nachfolgend unter Bezugnahme auf die Zeichnungen detailliert beschrieben. Hierbei zeigt:
- Fig. 1: hoch schematisch ein erfindungsgemäßes Infusionssystem,
- Fig. 2: hoch schematisch eine Spritzenpumpe zur Verwendung in einem erfindungsgemäßen Infusionssystem, und
- Fig. 3: hoch schematisch eine Schlauchpumpe zur Verwendung in einem erfindungsgemäßen Infusionssystem.

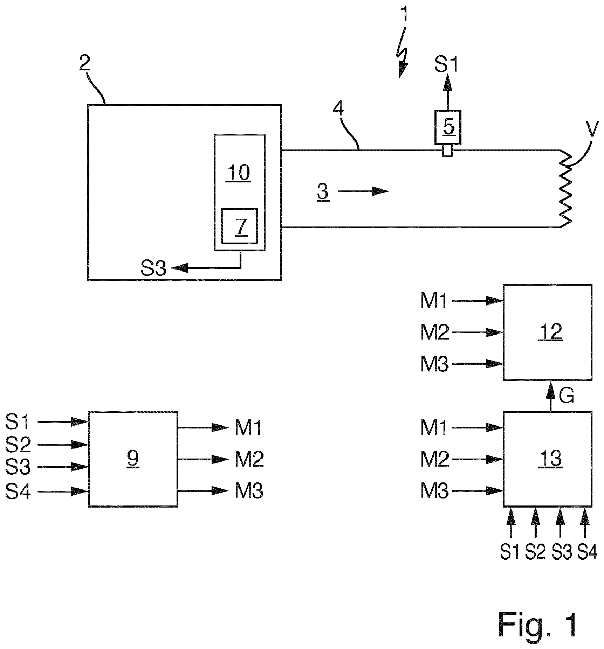

Fig. 1 zeigt hoch schematisch ein erfindungsgemäßes Infusionssystem 1 mit einer herkömmlichen Infusionspumpe 2, die dazu ausgebildet ist, eine zu verabreichende Flüssigkeit 3 in einer Flüssigkeitsleitung 4 zu fördern. Insoweit sei auch auf die einschlägige Fachliteratur verwiesen.

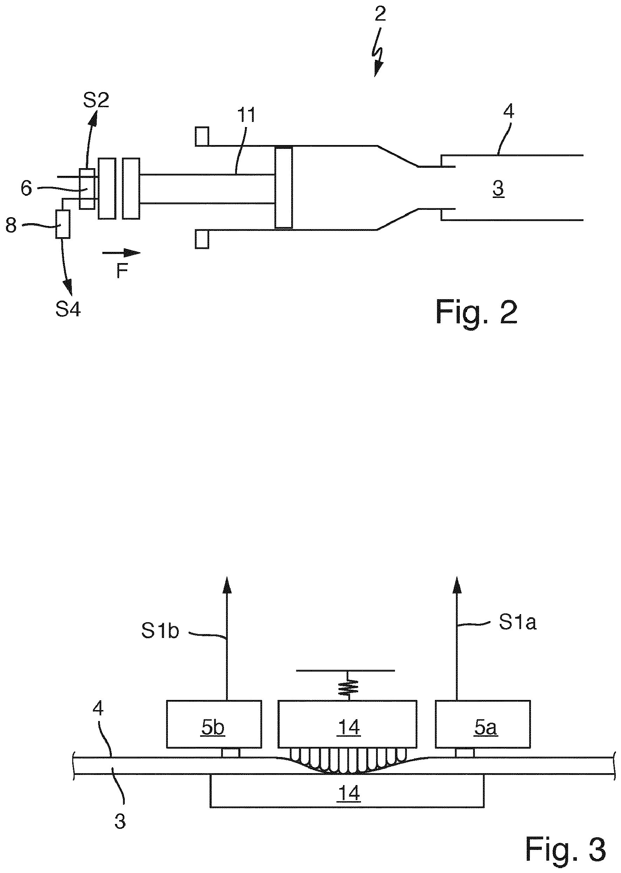

Das Infusionssystem 1 weist weiter Sensoren 5, 6, 7, 8 (siehe auch Fig. 2) zur Erzeugung zugehöriger Sensordaten S1, S2, S3, S4 auf. Die Sensordaten 5, 6, 7, 8 können analoge oder digitale Sensordaten sein.

Das Infusionssystem 1 weist weiter eine KI-Einheit 9 auf, die beispielsweise als Mikroprozessorsystem in Verbindung mit einer geeigneten Software verkörpert sein kann. Die KI-Einheit 9 ist dazu ausgebildet, eine erste Zielgröße M1, eine zweite Zielgröße M2 und eine dritte Zielgröße M3 in Abhängigkeit von der Anzahl von Sensordaten S1, S2, S3, S4 basierend auf einem statistischen Modell zu berechnen, wobei das statistische Modell zuvor mittels Trainingsdaten trainiert worden ist.

Die Anzahl von Sensoren weist auf: einen Drucksensor 5, der dazu ausgebildet ist, Sensordaten in Form von Druckdaten S1 der zu verabreichenden Flüssigkeit 3 in der Flüssigkeitsleitung 4 zu erzeugen, einen Kraftsensor 6 (siehe Fig. 2), der dazu ausgebildet ist, Sensordaten in Form von Kraftdaten S2 einer mittels der Infusionspumpe 2 erzeugten Pumpkraft F zu erzeugen, einen Motorstromsensor 7, der dazu ausgebildet ist, Sensordaten in Form von Motorstromdaten S3 eines elektrischen Antriebsmotors 10 der Infusionspumpe 2 zu erzeugen, und einen Positionssensor 8 (siehe Fig. 2), der dazu ausgebildet ist, Sensordaten in Form von Positionsdaten S4 eines Spritzenkolbens 11 der Infusionspumpe 2 zu erzeugen.

Die Anzahl von Zielgrößen weist auf: eine erste Zielgröße M1 in Form eines absoluten bzw. realen Drucks in der Flüssigkeitsleitung 4, eine zweite Zielgröße M2 in Form einer Wahrscheinlichkeit eines Verschlusses V der Flüssigkeitsleitung 4 und eine dritte Zielgröße M3 in Form eines Orts des Verschlusses V der Flüssigkeitsleitung 4.

Die KI-Einheit 9 kann dazu ausgebildet sein, die Zielgrößen M1, M2, M3 weiter in Abhängigkeit von Medikamentendaten, Patientendaten, Daten von weiteren Infusionspumpen, Therapiedaten, Umgebungsbedingungsdaten und/oder Vitalparameterdaten basierend auf dem statistischen Modell zu berechnen.

Das Infusionssystem 1 weist weiter eine Alarmeinheit 12 auf, die dazu ausgebildet ist, basierend auf den Zielgrößen M1, M2, M3 zu überprüfen, ob eine Alarmbedingung erfüllt ist, und einen Alarm zu erzeugen, falls die Alarmbedingung erfüllt ist.

Das Infusionssystem 1 weist weiter eine Korrelationsanalyseeinheit 13 auf, die dazu ausgebildet ist, basierend auf zumindest einem Teil der Sensordaten S1, S2, S3, S4 und/oder basierend auf zumindest einem Teil der Zielgrößen M1, M2, M3 Gewichtungsfaktoren G zu berechnen, wobei die Alarmeinheit 12 dazu ausgebildet ist, basierend auch auf den Gewichtungsfaktoren G zu überprüfen, ob die Alarmbedingung erfüllt ist. Die Alarmeinheit 12 ist dazu ausgebildet, die Infusionspumpe 2 beim Eintreten der Alarmbedingung zu stoppen.

Fig. 2 zeigt hoch schematisch eine Infusionspumpe 2 in Form einer herkömmlichen Spritzenpumpe zur Verwendung in einem erfindungsgemäßen Infusionssystem 1.

Der Kraftsensor 6 misst die resultierende Kraft F, welche auf eine Kolbenplatte des Spritzenkolbens 11 der Infusionspumpe 2 ausgeübt wird. Diese Kraft F setzt sich zusammen aus der über den Innenquerschnitt der Spritze wirkenden Kraft des (Flüssigkeits-) Drucks, d.h. der ersten Zielgröße M1, und überlagernden Störgrößen. Störgrößen sind unter anderem: positions- und temperaturabhängige Haft/Gleitreibungseffekte, patienten- und therapiespezifische Größen (Durchmesser des Zugangs, Mehrfachinfusion, Art des Zugangs, Bewegung durch Umlagerungen, etc.), physikalische Eigenschaften der zu fördernden Flüssigkeit (Viskosität, Temperatur, etc.) und Effekte verursacht durch Umgebungsbedingungen (Rettungswagen, Hubschrauber, Luftfeuchte, atmosphärischer Druck, etc.).

Im Fall eines Verschlusses steigt der Druck im Verlauf der Förderung an. Die Zunahme des Drucks ist eine Funktion aus der Länge der Flüssigkeitsleitung 4 bis zum Verschluss, d.h. dem Ort des Verschlusses bzw. der Zielgröße 3, des verwendeten Materials und Dimensionen des Überleitungssystems, der Temperatur sowie des geförderten Volumens ab dem Zeitpunkt des Verschlusses. Eine steigende Kraft F auf dem Antriebskopf resultiert in einem größeren Motorstrom. Dieser kann alternativ oder zusätzlich zum Kraftsensor mittels des optionalen Motorstromsensors 7 erfasst und ausgewertet werden.

Fig. 3 zeigt eine volumetrische Infusionspumpe bzw. Schlauchpumpe 14, bei der zwei Drucksensoren 5a und 5b entlang der Flüssigkeitsleitung 4 angeordnet sind, die jeweils eine Kraft messen, die durch den Druck in der Flüssigkeitsleitung 4 über deren Wand auf den jeweiligen Drucksensor 5a bzw. 5b Sensor ausgeübt wird. Aufgrund der Peristaltik sind die Drücke an der Upstream-Seite (Pumpeneingang/Vorratscontainer) und Downstream-Seite (Pumpenausgang/Patient) unterschiedlich, weswegen zwei unabhängige Drucksensoren 5a und 5b verwendet werden, die Sensordaten S1a bzw. S1b erzeugen.

Die mittels der Drucksensoren 5a bzw. 5b gemessene Kraft setzt sich zusammen aus der über den Innenquerschnitt der Flüssigkeitsleitung 4 wirkenden Kraft des Flüssigkeitsdrucks, d.h. der ersten Zielgröße, und den überlagernden Störgrößen. Störgrößen sind unter anderem: temperaturabhängige und nutzungsdauerabhängige Materialeigenschaften, Bedingungen an der Upstream-Seite (Höhe und Art des Behälters, verwendete Tropfkammer, etc.), patienten- und therapiespezifische Größen (Durchmesser des Zugangs, Mehrfachinfusion, Art des Zugangs, Bewegung durch Umlagerungen, etc.), physikalische Effekte des Pumpprinzips (Muster während der normalen Förderung, Muster durch mechanische Druckentlastung bei einem Verschluss, etc.), physikalische Eigenschaften der zu fördernden Flüssigkeit (Viskosität, Temperatur, etc.) und Effekte verursacht durch Umgebungsbedingungen (Rettungswagen, Hubschrauber, Luftfeuchte, atmosphärischer Druck, etc.).

Im Fall eines Verschlusses steigt der Druck der Flüssigkeit im Verlauf der Förderung an. Die Zunahme des Drucks ist eine Funktion aus der Länge der Leitung bis zum Ort des Verschlusses, d.h. der dritten Zielgröße, des verwendeten Materials und Dimensionen des Überleitungssystems, der Temperatur sowie des geförderten Volumens ab dem Zeitpunkt des Verschlusses.

Erfindungsgemäß kann zusätzlich zu den Sensordaten S1 bis S4 das Gesamtsystem eines Bettplatzes betrachtet werden, indem alle Daten der beteiligten Infusionspumpen, Informationen über die Medikation sowie verfügbare Patientendaten (z.B. von anderen Medizingeräten) in Relation gesetzt werden.

Beispiele für verwendbare Patientendaten sind z.B. Vitalparameter (Blutdruck, Pulsfrequenz, Atemfrequenz, Körpertemperatur, etc.) und weitere physiologische Parameter (Sauerstoffsättigung, Blutzuckerspiegel, Blutbild, Größe, Alter, Gewicht, Geschlecht, erbliche Faktoren, Vorerkrankungen, Diagnosen, etc.). Aus den Informationen über die Medikation können die Eigenschaften der geförderten Flüssigkeiten (Medikament und deren Kritikalität, Halbwertszeit/Wirkungsdauer, etc.) ermittelt werden.

Die Korrelationsanalyseeinheit 13 beschreibt Wechselwirkungen zwischen den gemessenen/ausgewerteten Größen und realisiert eine Korrelationsanalyse dieser Größen. Hierdurch kann u.a. eine frühere Erkennung eines Verschlusses ermöglicht werden, indem beispielsweise eine Korrelation zwischen einer Unterförderung eines Medikamentes und den anderen verfügbaren Patientendaten erkannt wird. Ein Beispiel wären abfallender Blutdruck und Herzfrequenz bei der Gabe von Adrenalin.

Die Alarmierung kann dann dahingehend intelligenter erfolgen, als dass die Empfindlichkeitsstufe der Verschlusserkennung bei einer vorliegenden Korrelation erhöht wird und dadurch früher als in den ursprünglichen Einstellungen vorgesehen alarmiert wird. Der umgekehrte Fall kann zudem bei unkritischen Medikamenten Alarme vermeiden, wodurch die Belastung des Anwenders durch unnötige Alarmierung verringert wird.

Die Veränderung von bestimmten Vitalparametern oder das Überschreiten von spezifischen Schwellwerten kann bei der Gabe von bestimmten Medikamenten/Ernährung auf einen Verschluss hindeuten. Für unkritische Medikamente/Flüssigkeiten könnten Alarmprioritäten heruntergesetzt werden, bzw. Alarmschwellen erhöht werden. Wechselwirkungen von verschiedenen Pumpen am gleichen Port können dediziert und bei der Verschlusserkennung berücksichtigt werden.

Das KI-System für den Bettplatz kann als zentrale Serveranwendung realisiert werden, welche auch eine Schnittstelle zu einer Cloud bereitstellen kann. Die gewonnen Daten können anschließend während der Weiterentwicklung in die Optimierung der Verschlusserkennung mit einfließen oder es können kundenspezifische Okklusionsmuster erstellt werden.

Ein spezieller Bettplatz ist der Transport in einem Rettungswagen bzw. einem Rettungsflugzeug, in denen Eingangsgrößen der Umgebungsbedingungen vorliegen. Hier können die Daten der Navigation verwendet werden, um z. B. Fliehkräfte, welche auf die Flüssigkeit wirken (Kurven/Steigungen), im System zu kompensieren.

## Patentansprüche

1. Infusionssystem (1), aufweisend:
- eine Infusionspumpe (2), die dazu ausgebildet ist, eine zu verabreichende Flüssigkeit (3) in einer Flüssigkeitsleitung (4) zu fördern,
- eine Anzahl von Sensoren (5, 6, 7, 8) zur Erzeugung zugehöriger Sensordaten (S1, S2, S3, S4), und
- eine KI-Einheit (9), die dazu ausgebildet ist, eine Anzahl von Zielgrößen (M1, M2, M3) in Abhängigkeit von der Anzahl von Sensordaten (S1, S2, S3, S4) basierend auf einem statistischen Modell zu berechnen, wobei das statistische Modell mittels Trainingsdaten trainiert worden ist.

2. Infusionssystem (1) nach Anspruch 1, **dadurch gekennzeichnet, dass**
- die Anzahl von Sensoren ausgewählt ist aus der Menge von Sensoren bestehend aus:
- Drucksensoren (5), die dazu ausgebildet sind, Sensordaten in Form von Druckdaten (S1) der zu verabreichenden Flüssigkeit (3) in der Flüssigkeitsleitung (4) zu erzeugen,
- Kraftsensoren (6), die dazu ausgebildet sind, Sensordaten in Form von Kraftdaten (S2) einer mittels der Infusionspumpe (2) erzeugten Pumpkraft (F) zu erzeugen,
- Motorstromsensoren (7), die dazu ausgebildet sind, Sensordaten in Form von Motorstromdaten (S3) eines elektrischen Antriebsmotors (10) der Infusionspumpe (2) zu erzeugen, und
- Positionssensoren (8), die dazu ausgebildet sind, Sensordaten in Form von Positionsdaten (S4) eines Spritzenkolbens (11) der Infusionspumpe (2) zu erzeugen.

3. Infusionssystem (1) nach einem der vorhergehenden Ansprüche, **dadurch gekennzeichnet, dass**
- die Anzahl von Zielgrößen ausgewählt ist aus der Menge von Zielgrößen bestehend aus:
- einem absoluten Druck (M1) in der Flüssigkeitsleitung (4),
- einer Wahrscheinlichkeit (M2) eines Verschlusses (V) der Flüssigkeitsleitung (4) und
- einem Ort (M3) des Verschlusses (V) der Flüssigkeitsleitung (4).

4. Infusionssystem (1) nach einem der vorhergehenden Ansprüche, **dadurch gekennzeichnet, dass**
- die KI-Einheit (9) dazu ausgebildet ist, die Anzahl von Zielgrößen (M1, M2, M3) weiter in Abhängigkeit von Medikamentendaten, Patientendaten, Daten von weiteren Infusionspumpen, Therapiedaten, Umgebungsbedingungsdaten und/oder Vitalparameterdaten basierend auf dem statistischen Modell zu berechnen.

5. Infusionssystem (1) nach einem der vorhergehenden Ansprüche, **dadurch gekennzeichnet, dass**
- das Infusionssystem (1) eine Alarmeinheit (12) aufweist, die dazu ausgebildet ist, basierend auf der Anzahl von Zielgrößen (M1, M2, M3) zu überprüfen, ob eine Alarmbedingung erfüllt ist, und einen Alarm zu erzeugen, falls die Alarmbedingung erfüllt ist.

6. Infusionssystem (1) nach Anspruch 5, **dadurch gekennzeichnet, dass**
- das Infusionssystem (1) eine Korrelationsanalyseeinheit (13) aufweist, die dazu ausgebildet ist, basierend auf zumindest einem Teil der Anzahl von Sensordaten (S1, S2, S3, S4) und/oder basierend auf zumindest einem Teil der Anzahl von Zielgrößen (M1, M2, M3) Gewichtungsfaktoren (G) zu berechnen, wobei die Alarmeinheit (12) dazu ausgebildet ist, basierend auch auf den Gewichtungsfaktoren (G) zu überprüfen, ob die Alarmbedingung erfüllt ist.

7. Infusionssystem (1) nach Anspruch 5 oder 6, **dadurch gekennzeichnet, dass**
- die Alarmeinheit (12) dazu ausgebildet ist, die Infusionspumpe (2) beim Eintreten der Alarmbedingung zu stoppen.
